(19) Europäisches Patentamt
European Patent Office
Office européen des brevets

(11) Publication number: 0 279 150 A1

# (12) EUROPEAN PATENT APPLICATION

(21) Application number: 87850388.7

(22) Date of filing: 16.12.87

(51) Int. Cl.4: C07D 311/58 , A61K 31/35

The title of the invention has been amended (Guidelines for Examination in the EPO, A-III, 7.3).

(30) Priority: 19.12.86 SE 8605504

(43) Date of publication of application: 24.08.88 Bulletin 88/34

(84) Designated Contracting States: ES GR

(71) Applicant: ASTRA LAKEMEDEL AKTIEBOLAG
S-151 85 Södertälje(SE)

(72) Inventor: Häll, Hakan Olof
Lingonvägen 20
S-155 00 Nykvarn(SE)
Inventor: Johansson, Lars George
Liljewalchsgatan 9
S-151 45 Södertälje(SE)
Inventor: Thorberg, Seth Olov
Gullvivsstigen 42
S-153 00 Järna(SE)

(74) Representative: Hjertman, Ivan T. et al
AB ASTRA Patent and Trade Mark Department
S-151 85 Södertälje(SE)

## (54) 5-Oxy-substituted-3-aminochroman compounds, processes for their preparation, pharmaceutical compositions containing them and methods of treatment therewith.

(57) A compound of the formula

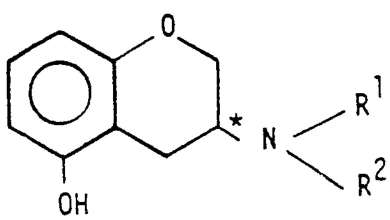

I

wherein $R^1$ is a saturated or unsaturated alkyl group having 1-5 carbon atoms or a phenylalkyl group, whereby the phenyl ring may be substituted or unsubstituted and the alkyl having 1-4 carbon atoms, $R^2$ is hydrogen or an alkyl group having 1-5 carbon atoms, or $R^1$ and $R^2$ together form a 5-or 6-membered ring containing 1 or 2 heteroatoms selected from N, O and S, and enantiomers and physiologically acceptable salts thereof, processes for preparation of said compounds, pharmaceutical preparations containing said compounds, use of and method of treatment of disorders in CNS by using said compounds and intermediates.

EP 0 279 150 A1

# Novel Chroman Derivatives

## Field of the Invention

The present invention relates to novel 5-hydroxy-3-aminochromans enantiomers and salts thereof, to processes for the preparation of such compounds as well as new intermediates useful in the preparation of 5-hydroxy-3-aminochromans. The invention further relates to pharmaceutical preparations containing said compounds and the use of said compounds in therapy.

An object of the invention is to provide compound for therapeutic use, especially compound having a therapeutic activity via the central nervous system (CNS). A further object is to provide compounds having a selective effect on the 5-hydroxy-tryptamine receptors in mammals including man.

## Prior Art

In J.Med.Chem. vol. 27, 1340-1343 (1984), 6,7-dihydroxy-3-aminochroman is disclosed and said to possess dopaminergic activity in the central nervous system.

In EP 0 041 488 tetralines of the formula

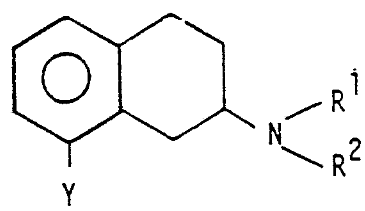

wherein Y is hydroxy and $R^1$ is alkyl having 1 to 8 carbon atoms, benzyl or phenethyl, and $R^2$ is hydrogen or alkyl having 1 to 5 carbon atoms, are described. Such compounds are indicated to have effect on the 5-hydroxy tryptamine neurons in mammals.

## Disclosure of the Invention

It has been found that compounds of the formula I

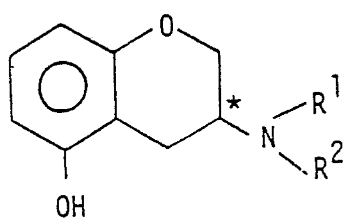

I

wherein $R^1$ is a saturated or unsaturated alkyl group having 1-5 carbon atoms or a phenylalkyl group whereby the phenyl ring may be substituted or unsubstituted and the alkyl having 1-4 carbon atoms, $R^2$ is hydrogen or an alkyl group having 1 to 5 carbon atoms, or $R^1$ and $R^2$ together form a saturated or unsaturatged 5-or 6-membered ring containing 1 to 2 heteroatoms selected from N, O and S and enantiomers as well as physiologically acceptable salts thereof, possess unexpected pharmacological properties rendering them useful in therapy in the central nervous system, especially in the serotonergic system and closely related systems.

The invention thus provides compound, enantiomers and physiologically acceptable salts thereof, which are useful in therapeutic treatment of 5-hydroxy-tryptamine mediated states and disorders such as depression, anxiety, anorexia, senile dementia, Alzheimer's disease, hypertension, termoregulator and sexual disturbances. A further aspect of the invention is related to the use of the compounds and salts

thereof in pain control.

Alkyl groups in formula I representing straight, branched and cyclic alkyl groups having 1-5 carbon atoms e.g. methyl, ethyl, n-propyl, i-propyl, n-butyl, i-butyl, t-butyl, n-pentyl, i-pentyl, t-butyl, cyclopropyl, cyclobutyl, cyclopentyl, allyl, butenyl or pentenyl.

Possibly substituted phenylalkyl group in formula I representing substituted or unsubstituted phenylalkyl, wherein the alkyl group is a straight or branched alkyl having 1-4 carbon atoms and the aromatic ring may be substituted by one or more of fluoro, chloro, bromo, trifluoromethyl, methyl, ethyl, hydroxy, methoxy or ethoxy preferably in meta and/or para positions.

Examples of suitable 5-or 6-membered ring structures formed by $R^1$, $R^2$ and the nitrogen atoms and containing N, O and S are piperazine and morpholine.

The compounds of the invention have an asymmetric carbon atom in the oxygen containing ring moiety i.e. the ring carbon atom adjacent to the nitrogen atom. Thus, the compounds exist as two optical isomers i.e. enantiomers. Both the pure enantiomers, racemic mixtures (50% of each enantiomer) and unequal mixtures of the two are within the scope of the present invention. The therapeutic properties of the compounds may to a greater or lesser degree be ascribed to either or both of the two enantiomers occuring.

Both organic and inorganic acids can be employed to form non-toxic physiologically acceptable acid addition salts of the compounds of this invention. Illustrative acids are sulfuric, nitric, phosphoric, oxalic, hydrochloric, hydrobromic, citric, acetic, lactic, tartaric, pamoic, ethanedisulfonic, sulfamic, succinic, cyclohexylsulfamic, fumaric, maleic and benzoic acid. These salts are readily prepared by methods known in the art.

Preferred compounds are those wherein $R^1$ is an alkyl group having 2-3 carbon atoms and $R^2$ are hydrogen or an alkyl group having 2-3 carbon atoms. Further preferred are compounds wherein $R^1$ is n-propyl and $R^2$ are hydrogen, ethyl or n-propyl.

## Intermediates

Some of the intermediates of starting materials mentioned above and the preparation thereof are known. However, certain intermediates of starting materials are novel and constitute a further aspect of the invention. Thus, in one aspect the invention is related to novel compounds of the formula

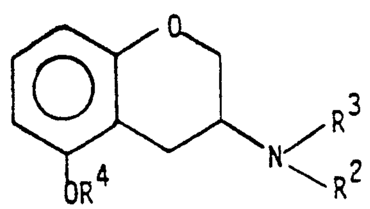

IA

wherein $R^4$ is hydrogen or saturated or unsaturated alkyl containing 1-5 carbon atoms or a phenylalkyl whereby the phenyl ring may be substituted or unsubstituted and the alkyl having 1-4 carbon atoms, $R^3$ is hydrogen or saturated or unsaturated alkyl containing 1-5 carbon atoms or a phenylalkyl whereby the phenyl ring may be substituted or unsubstituted and the alkyl having 1-4 carbon atoms, $R^2$ is hydrogen or alkyl containing 1-5 carbon atoms or $R^2$ and $R^3$ together form a 5-or 6-membered ring containing 1 or 2 heteroatoms selected from N, O and S with the proviso that when $R^4$ is hydrogen then $R^3$ is hydrogen as well as to enantiomers and salts of said compounds, and to the methods for preparing said compounds or salts. Preferred intermediates are those wherein $R^4$ is a methyl group.

Some of the compounds of formula IA have activity on the 5-HT system in CNS and are useful as thereapeutic agents in the deceases defined for the compounds of formula I.

## Methods of Preparation

The compounds of the invention may be prepared by one of the following methods constituting a further aspect of the invention.

a) Cleaving of an ether of the formula

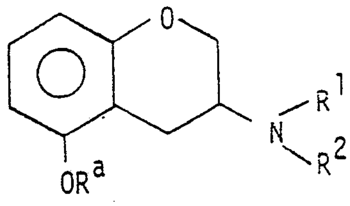

II

wherein $R^a$ represents a hydrocarbon residue, preferably an alkyl group having 1-5 carbon atoms, a benzyl or allyl group and $R^1$ and $R^2$ are as defined in formula I to form a compound of formula I. The cleavage may be carried out by treating the compound of formula II with an acidic nucleophilic reagent such as aqueous HBr, or HI, $HBr/CH_3COOH$, $BBr_3$, $AlCl_3$, pyridine-HCl or $(CH_3)_3SiI$, with a basic nucleophilic reagent such as $CH_3C_6H_4\text{-}S^-$ or $C_2H_5\text{-}S^-$.
When $R^a$ is a benzyl group the cleavage may also be carried out by reduction, preferably with hydrogen using Pd or $PtO_2$ as catalyst.

b) N-alkylation of a compound of formula III

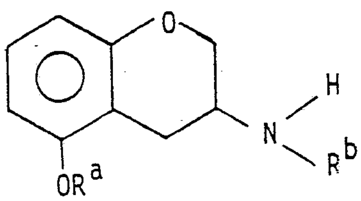

III

wherein $R^a$ is as defined in formula II, $R^b$ is either $R^1$ or $R^2$, and $R^1$ and $R^2$ are as defined under formula I, into a compound of formula II, by alkylation of the nitrogen atom with an appropriate alkylating agent. Thus, the starting compound wherein $R^b$ is $R^2$ may be treated with an alkyl, benzyl or phenethyl halide or tosylate $R^1X'$, wherein X' represents Cl, Br, I or

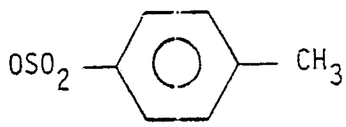

in an organic solvent such as acetonitrile or acetone and in the presence of a base such as $K_2CO_3$ or NaOH, or said starting compound may be treated with an carboxylic acid $NaBH_4$ complex $R^eCOOHNaBH_4$, wherein $R^e$is defined by the relation $R^e\text{-}CH_2\text{-}$equals $R^1$. To the formation of a compound of formula I wherein at least one of $R^1$ and $R^2$ is $CH_3$, the alkylation reaction may be caried out by treatment of the compound of formula III with a formaldehyde - $Na(CN)BH_3$ mixture, or with formaldehyde and formic acid.

c) Reduction of an amide of the formula

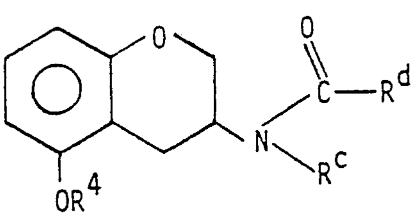

IV

wherein $R^4$ is hydrogen or $R^*$, $R^c$ is hydrogen or $R^*$, whereby $R^*$ is defined as in formula I and $R^d$ is hydrogen or an alkyl group having 1 to 4 carbon atoms for instance by treatment with a hydride reducing agent such as $LiAlH_4$ in ether or tetrahydrofuran or $BH_3$ in tetrahydrofuran, to the formation of a compound of formula I or IA.

d) Catalytic reduction of a compound of the formula

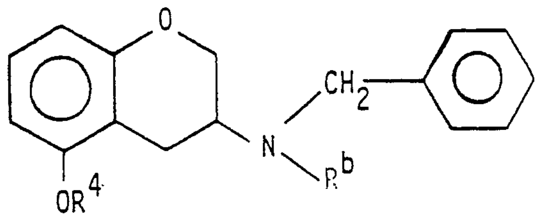

V

wherein $R^4$ and $R^b$ are as defined above, to the formation of a compound of formula III by treatment with e.g. $H_2$/Pd. With appropriate meanings of $R^4$ and $R^b$ the product obtained also satisfies formula I or IA.

e) Converting a compound of the formula

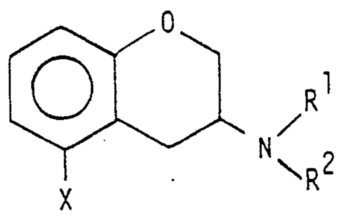

VI

wherein X represents $SO_3H$, Cl or $NH_2$ and $R^1$ and $R^2$ are defined as above by substitution of the group X to a hydroxy group to formation of a compound of formula I. When X is $SO_3H$ or Cl said reaction may be carried out by treatment with a strong alkali under heating, suitably with an alkali melt such as KOH when X is $SO_3H$, and with a strong aqueous alkali such as NaOH or KOH when X is Cl.
When X is $NH_2$ the reaction may be carried out by treatment with aqueous nitrous acid to the formation of an intermediate diazonium compound which is then subjected to hydrolysis in water.

f) Converting an enamine with either a $C_1$-$C_2$ or a $C_2$-$C_3$ double bound or an imine base (without $R^2$) or immonium salt (e.g. $ClO_4$-or $BF_4$-) with a $C_2$-N double bound of the formula

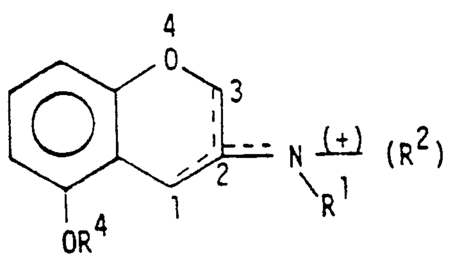

VII

wherein $R^4$, $R^1$ and $R^2$ are as defined above by reduction to a compound of formula I or IA, preferably by catalytic hydrogenation using $PtO_2$ or Pd as a catalyst.

Free bases formed may subsequently be converted into their acid addition salts, and acid addition salts formed may subsequently be converted into the corresponding bases or other acid addition salts.

## Preparation of Intermediates

The novel intermediates with the general formula IA may be obtained by the methods described below or by methods known in the art.

i) the starting material for method a) according to formula II may inter alia be prepared by the following method:

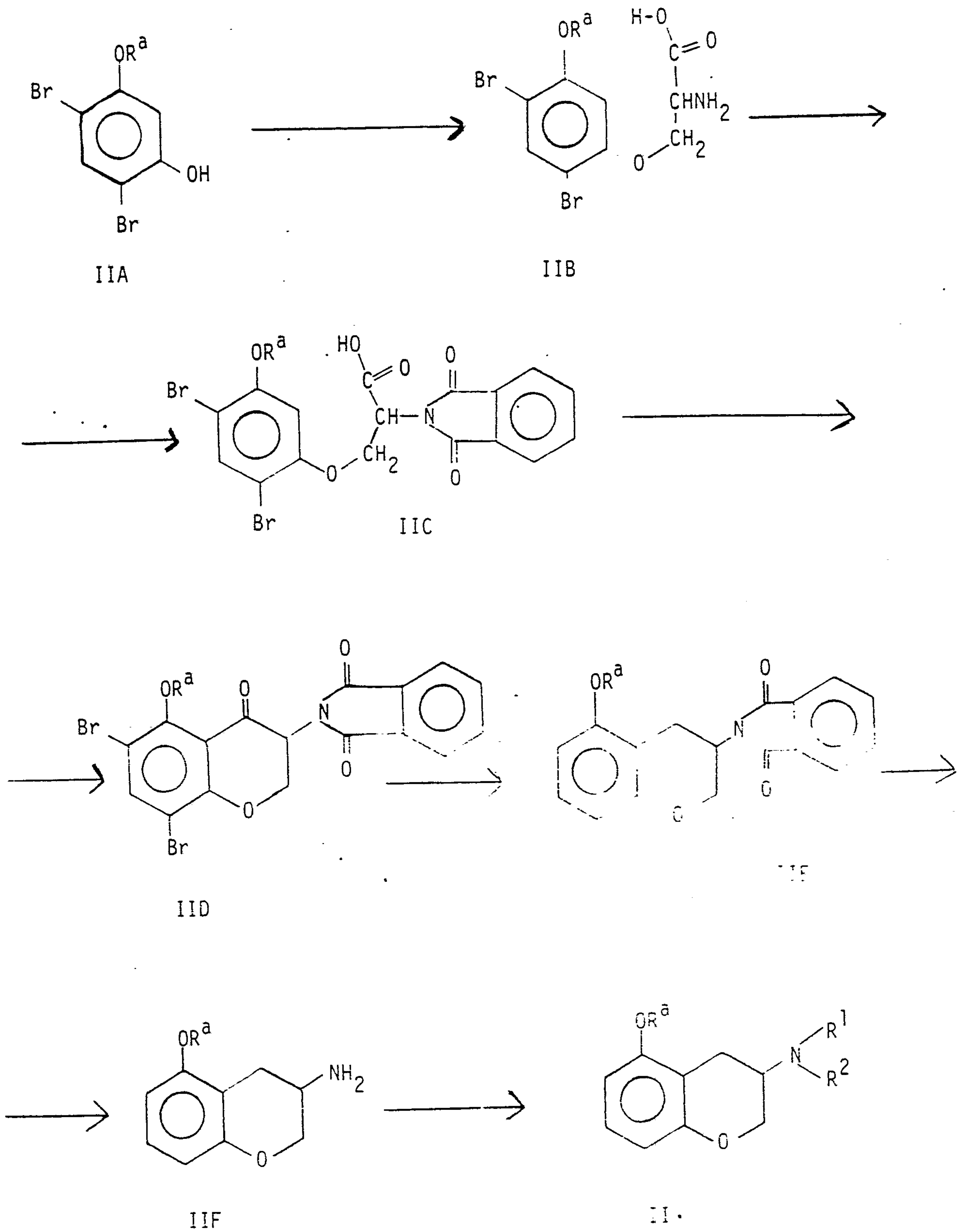

IIA IIB IIC IID IIE IIF II

A compound of the formula IIA is reacted by phenolic alkylation with L-or D-chlorocystein to the formation of a compound IIB, which is reacted with a phtalic acid anhydride to the formation of a compound of formula IIC. Compound IIC is cyclizised by a Friedel-Craft acylation preferably by using aluminium chloride and thionyl chloride ($AlCl_3$-$SOCl_2$) to a compound of the formula IID, which is reduced by catalytic hydrogenation preferably using $H_2$-Pd to the formation of a compound IIE. Then compound IIE is

deftaloylated by using hydrazine ($H_2N-NH_2$) to the formation of the L-or D-form of compound IIF. Then a secondary or tertiary amine is desired, compound IIF is alkylated preferably with an alkylhalide such as alkylbromide to a compound II.

The intermediate of the formula III in form of a primary amine may be prepared by the following methods. Corresponding secondary or tertiary amine included in formula III may be prepared from the primary amine of formula III by alkylation with an alkylhalide such as alkylbromide to formation of the amine desired or by methods described below.

ii)

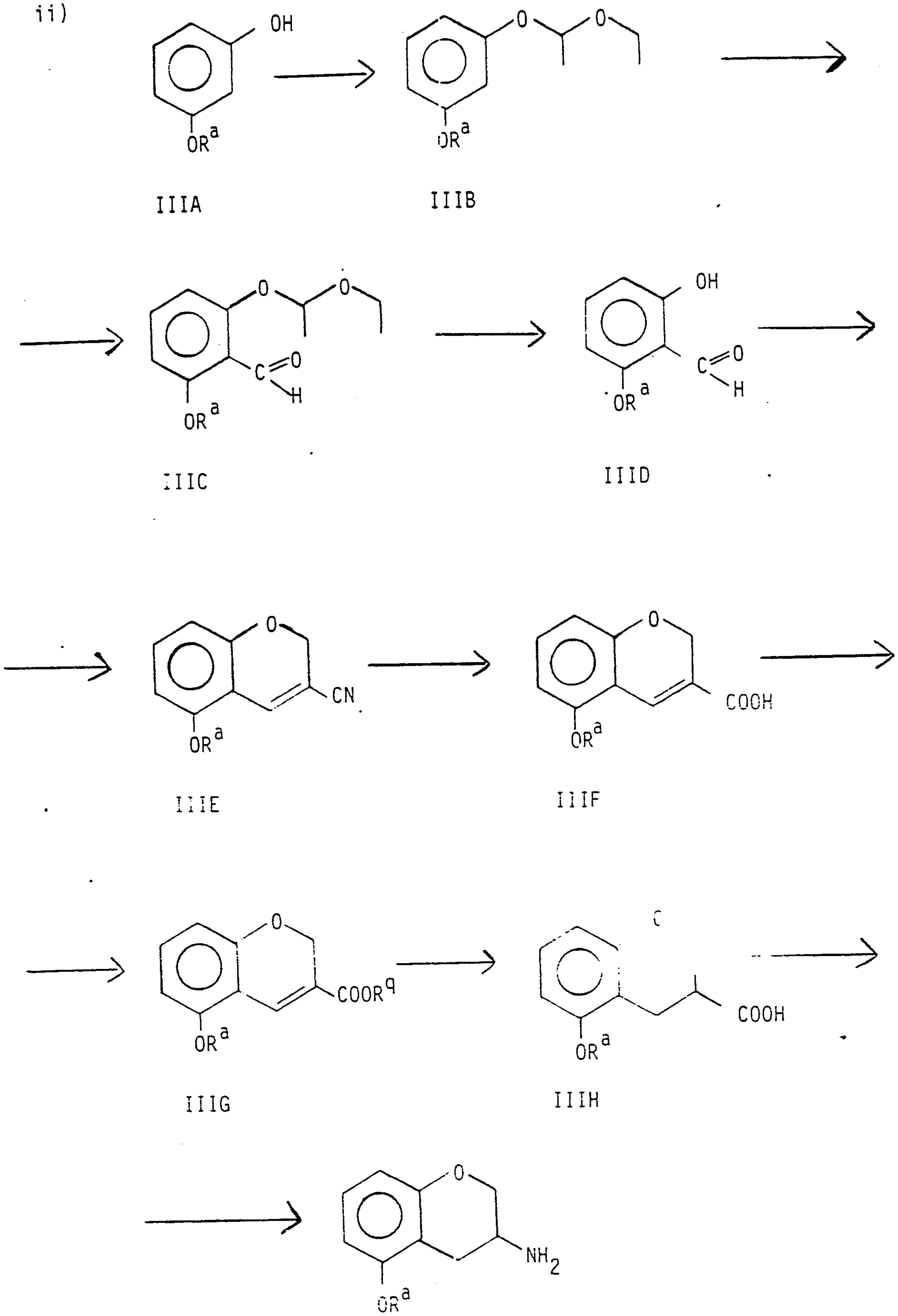

IIIA IIIB

IIIC IIID

IIIE IIIF

IIIG IIIH

II

A compound of formula IIIA is reacted with alkylvinylether such as ethylvinylether in presence of trichloroacetic acid as catalyst to the formation of a compound of formula IIIB, which is reacted with butyllithium in dimethyl formamide (BuLi/DMF) to the formation of a compound of formula IIIC, which is deprotected by using HCl. Compound IIID is reacted by 1,4-addition of acrylonitrile in DABCO® followed by an intramolecular condensation to formation of a compound of formula IIIE, the compound is then alkali hydrolyzed to a compound IIIF and estrificated with $R^qOH$, wherein $R^q$ is a suitable alkyl group, followed by reduction preferably with $H_2$,Pd to the formation of a compound IIIH. Compound IIIH is then reacted by a Curtius rearrangement preferably by using diphenylphosphorazidate in presence of trialkylamin, followed by addition of benzylalcohol and reduction ($H_2$,Pd) to the formation of a primary amine of formula III.

iii)

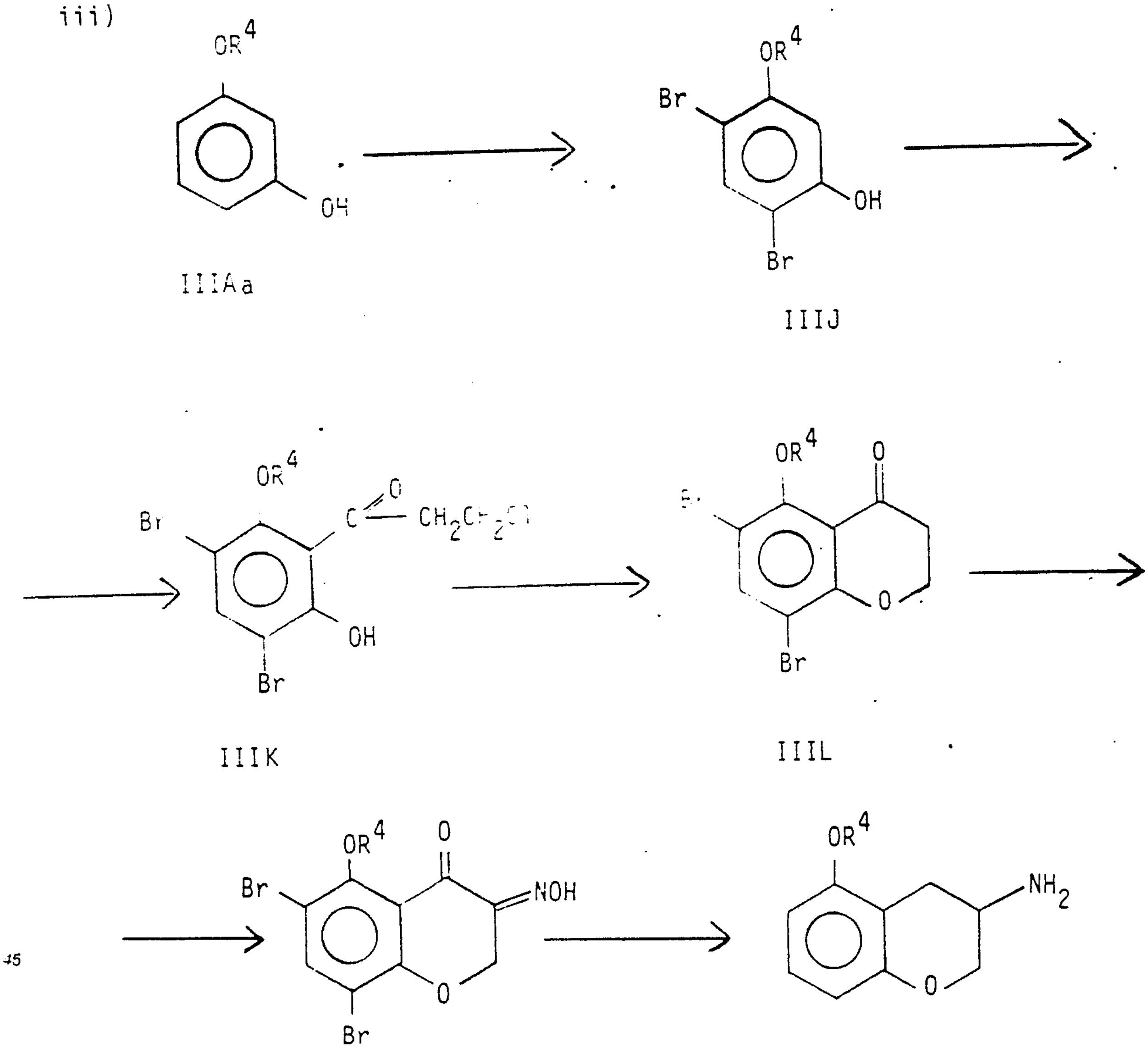

IIIAa IIIJ

IIIK IIIL

IIIM IA

A compound of formula $IIIA_a$ is brominated to the formation of a compound of formula IIIJ which is reacted by Friedel-Craft acylation for instance with $ClCOCH_2CH_2Cl$ to formation of a compound of formula IIIK. The compound IIIK is then cyclizised in presence of potassiumcarbonate to a compound IIIL which is then treated with tert-butyl nitrite in presence of $t\text{-}BuO^-K^+$ to the formation of a compound of formula IIIM followed by reduction, preferably using Pd/$BaSO_4$, $LiAlH_4$, to the formation of a primary amin of formula IA. Alternatively, compound IIIL may be reacted by a Neber reaction using for instance iso-$C_3H_7ONa$ and $LiAlH_4$

to the formation of the compound of formula IA, directly.

iv)

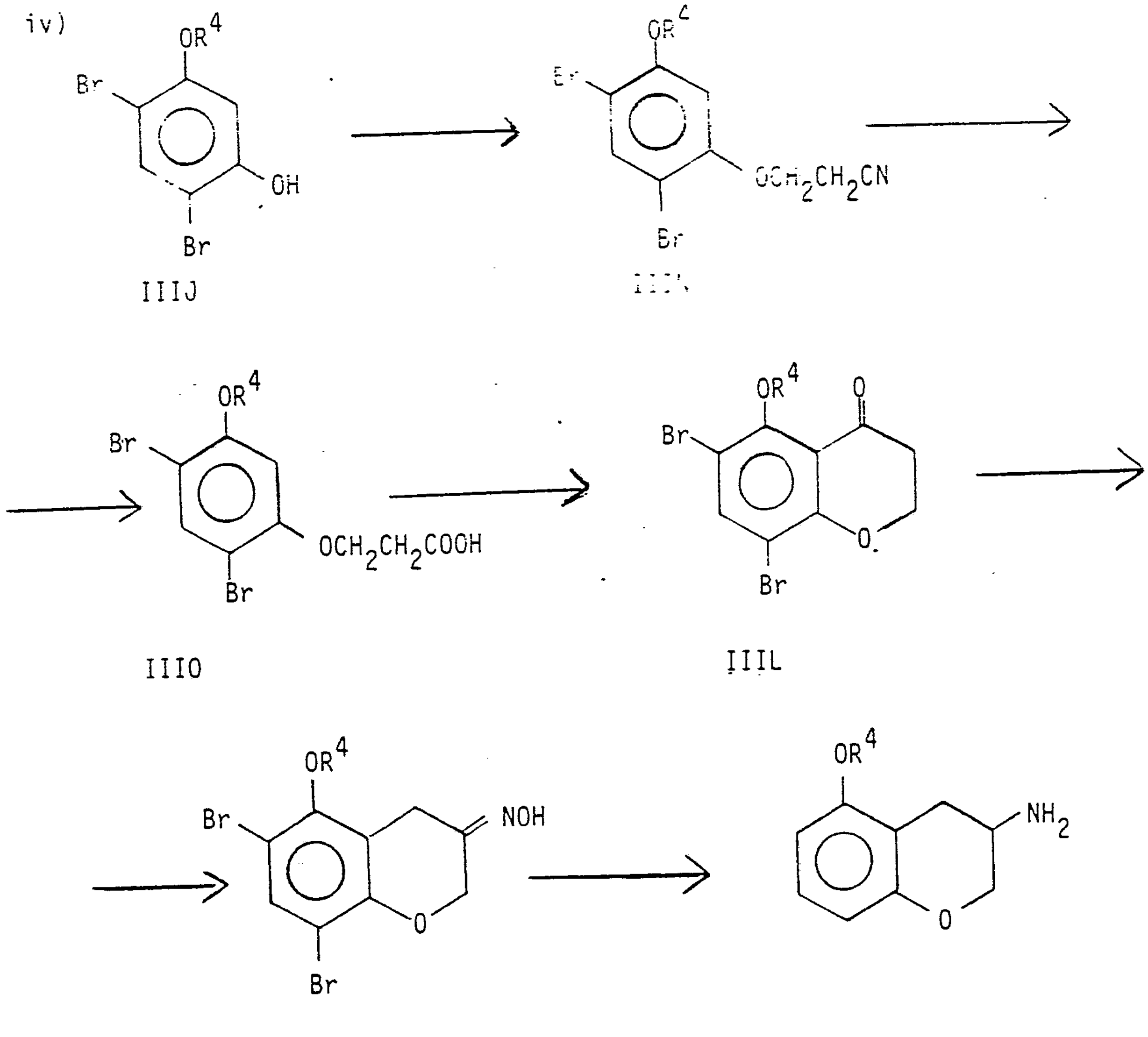

IIIJ

IIIN

IIIO

IIIL

IIIM

IA

A compound of formula IIIJ is reacted by an 1,4-addition with one equivalent vinylnitril to the formation of compound IIIN, which is hydrolyzed (NaOH/KOH) to compound IIIO. Compound IIIO is reacted by a Friedel-Craft acylation using $PCl_5$, $AlCl_3$ to the formation of a compound of formula IIIL which subsequently is reacted with tert-butylnitrile and tert.$BuO^-K^+$ followed by reduction or by Neber reaction described in method iii) to formation of a primary amine of formula IA.

v)

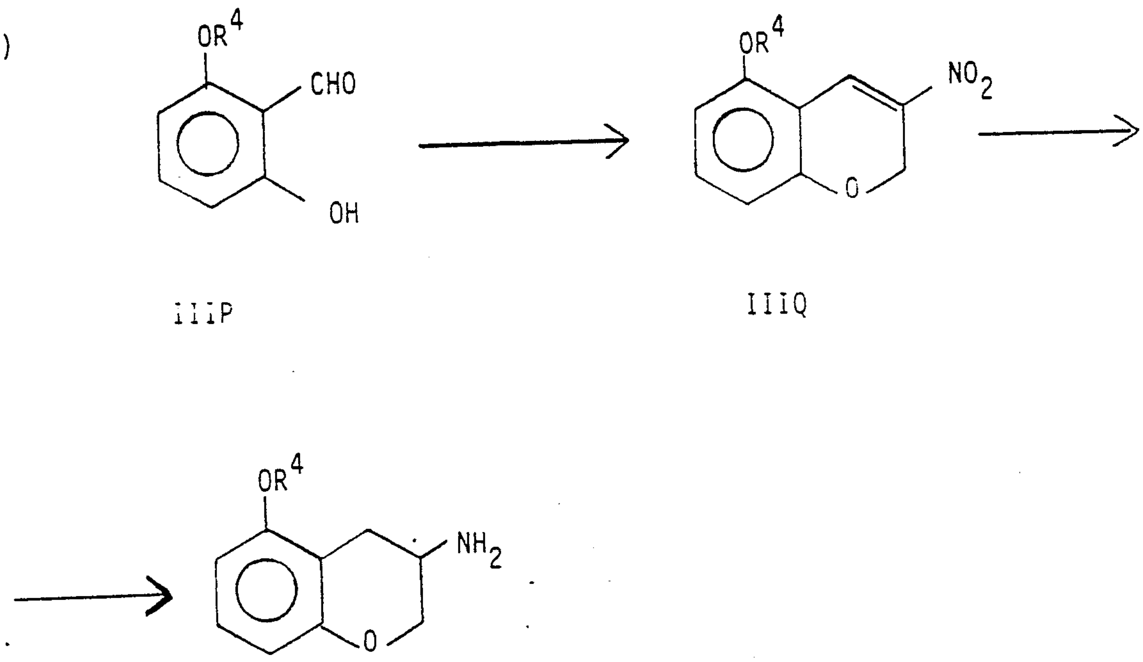

IIIP

IIIQ

IA

A compound of the formula IIIP is reacted with one equivalent chloroethylnitrite ($ClCH_2CH_2NO_2$) to the formation of a compound of formula IIIQ which is then catalyitic hydrogenated to the formation of the primary amine of formula IA.

vi)

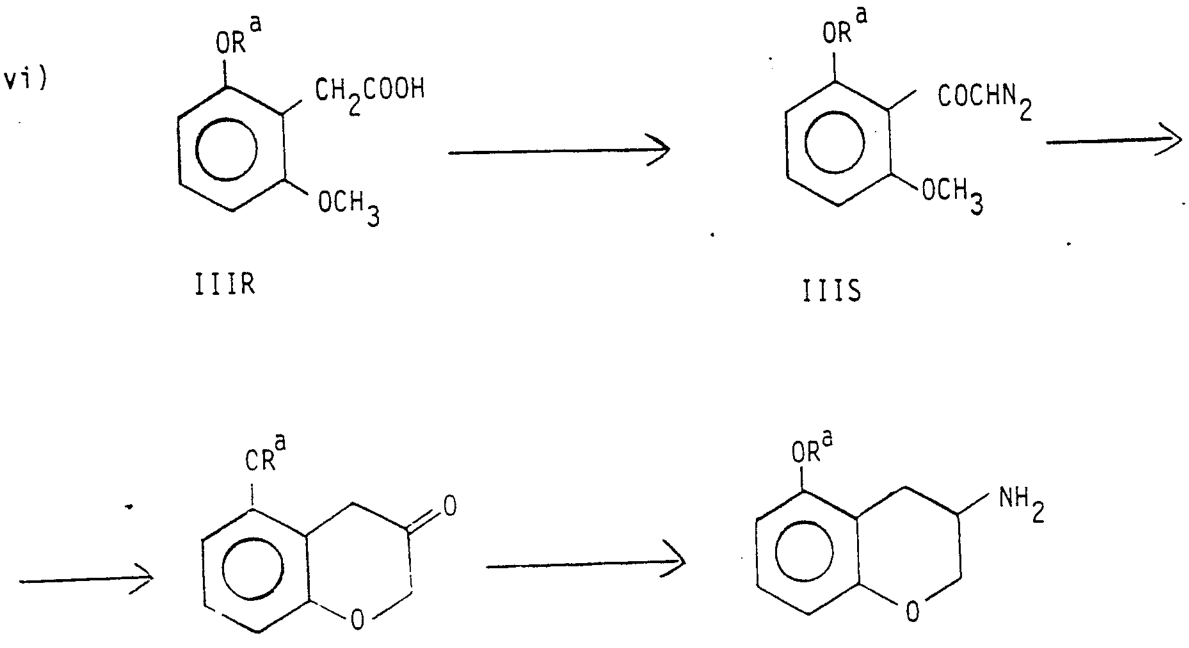

IIIR

IIIS

IIIT

III

A compound of the formula IIIR is diazotated using diazomethane in presence of tionylchloride to the formation of a compound of formula IIIS. which is reacted with $C_2H_5OBF_3$ to formation of compound IIIT which is reductive aminated to formation of the primary amine of formula III.

vii)

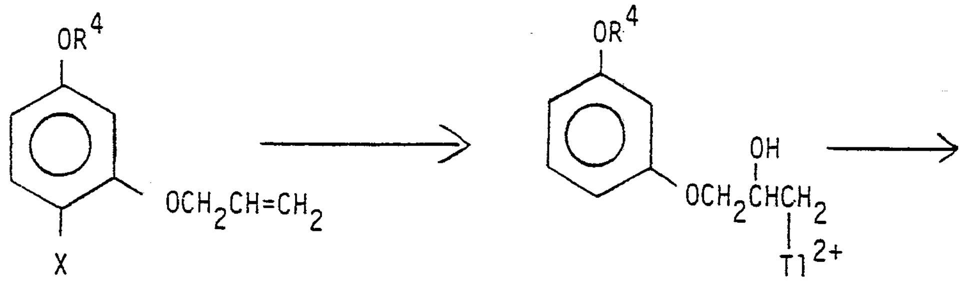

IIIO IIIU

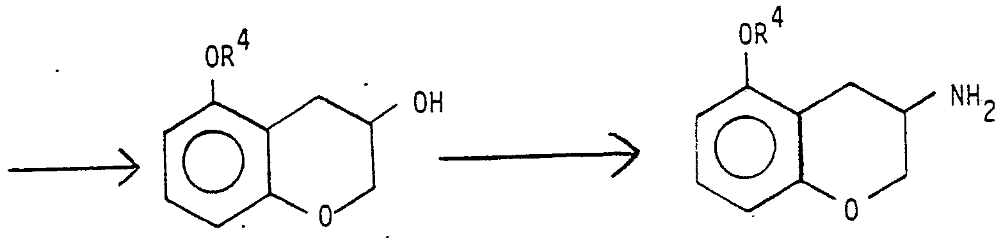

IIIY IA

A compound of formula IIIO is treated with a $TL^{3+}$ salt during alkaline conditions forming the adduct IIIU which is cyclizised to IIIY and transferred to IA via tosylate and azide.

viii)

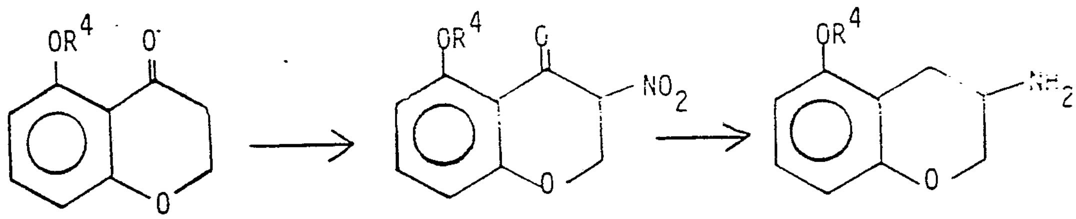

VIIIA VIIIB IA

A compound of formula VIIIA is reacted with a nitrate to the formation of a compound of formula VIIIB, which is reduced to a primary amine of formula IA.

ix)

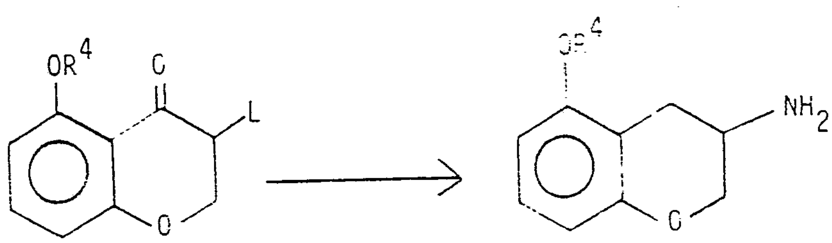

VIIIC IA

The compound of the formula VIIIC wherein L is a leaving group such as chloride, bromide or tosylate is reacted via a reaction with potassiumphthalimide and subsequent formation of a primary amine of formula IA by treatment with hydrazine and $H_2$-Pd.

x) The starting material for method c) according to the formula IV may be prepared by the following methods:

x)

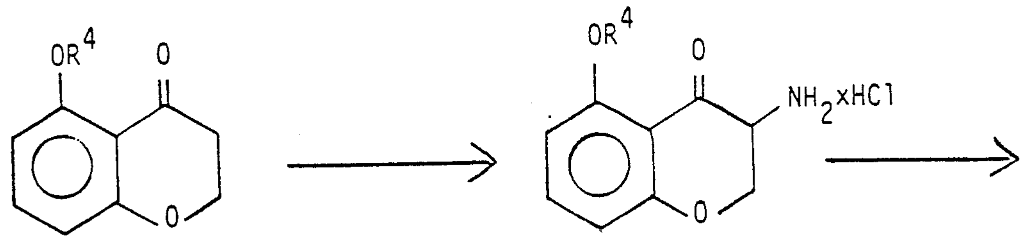

IVA IVB

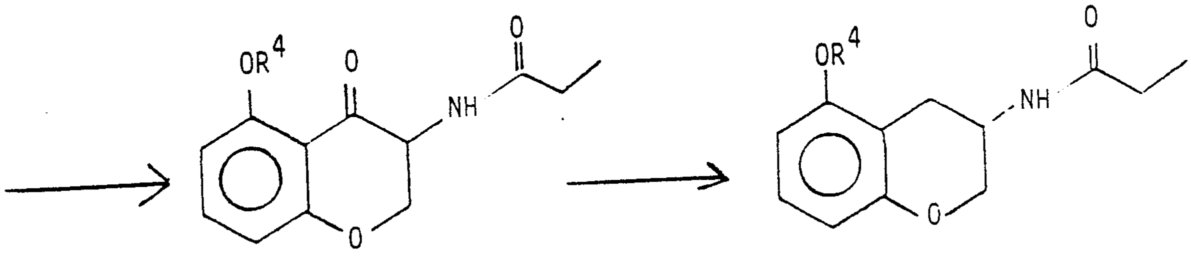

IVC IV

A compound of formula IVA is reacted by a Neber rearrangement in three steps using for instance in step a) $NH_2OH x HCl$, in step b) tosylchloride and in step c) $C_2H_5ONa$. HCl to formation of a compound of formula IVB, which is then acylated with an acylhalide to compound IVC. Compound IVC is catalytic hydrogenated ($H_2$ PD) to formation of a compound of formula IV.

xi)

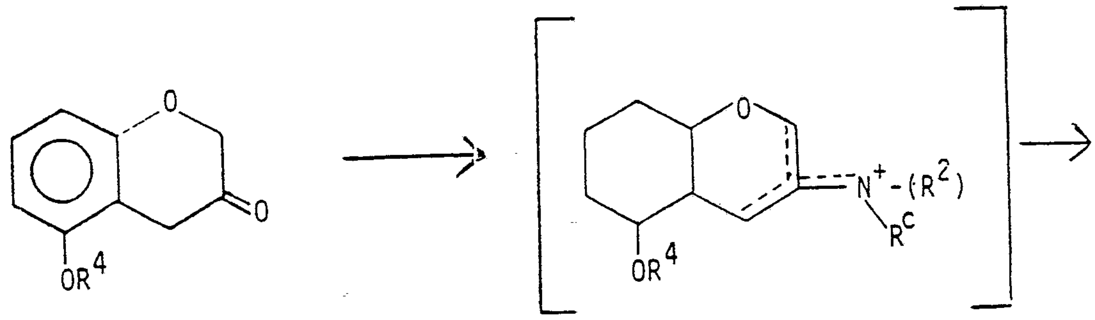

IIIT VII

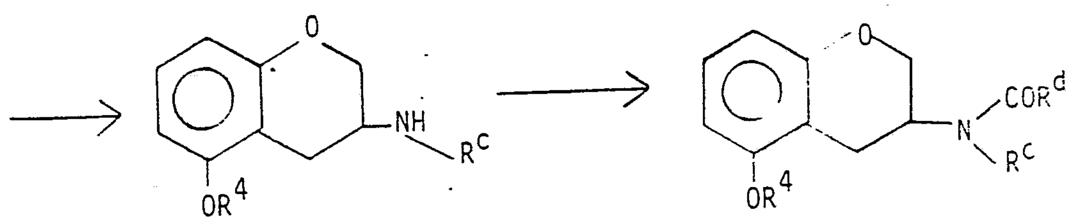

IA IV

A compound of the formula IIIT is reacted with an amine such as $R^{C}H_2$ in presence of acetic acid and molecular sieves or methylamine hydrochloride in presence of NaOH to the formation of an intermediate imine, which is reduced by catalytic reduction preferably using $PtO_2$ or Pd to the formation of a primary or a secondary amine of formula IA. When a tertiary amine is desired, compound IA is then acylated with a carboxylic acid chloride in the presence of triethylamine and subsequently reduced to the formation of the compound of the formula IV.

xii) The starting material for method d) according to formula V may be prepared as described above or by benzylation of a primary amine of formula IA with a benzylhalide such as benzylbromide or benzylchloride followed by subsequent reduction with $LiAlH_4$.

xiii)The starting material for method f) according to formula VII is prepared as described in method xi) above.

## Pharmaceutical Preparations

Pharmaceutical preparations of the compounds of the invention constitute a further aspect of the invention.

In clinical practice the compounds of the present invention will normally be administered orally, rectally, or by injection, in the form of pharmaceutical preparations comprising the active ingredient either as a free base or as a pharmaceutically acceptable non-toxic, acid addition salt, e.g. the hydrochloride, lactate, acetate, sulfamate, and the like in association with a pharmaceutically acceptable carrier.

Accordingly, terms relating to the novel compounds of this invention, whether generically or specifically, are intended to include both the free amine base and the acid addition salts of the free base, unless the context in which such terms are used, e.g. in the specific examples, would be inconsistent with the broad concept. The carrier may be a solid, semisolid or liquid diluent or capsule. These pharmaceutical preparations constitute a further aspect of this invention. Usually the active substance will constitute betwen 0.1 and 99% by weight of the preparation, more specifically between 0.5 and 20% by weight for preparation intended for injection and betwen 0.2 and 95% by weight for preparations suitable for oral adminstration.

Pharmaceutical preparations containing a compound of the invention in a solid form of dosage units for oral applications may preferably contain between 2 and 95% by weignt of the active substance. In such

preparations the selected compound may be mixed with a solid fine grain carrier, e.g. lactose, saccharose, sorbitol, mannitol, starches such as potato starch, corn starch or amylopectin, cellulose derivatives, or gelatin and lubricants such as magnesium stearate, calcium stearate, polyethylene glycol waxes, and the like, and then compressed to form tablets. If coated tablets are required, the cores, prepared as described above, may be coated with a concentrated sugar solution which may contain, e.g. gum arabic, gelatin, talcum, titanium dioxide, and the like. Alternatively the tablet can be coated with a lacquer dissolved in a readily volatile organic solvent or mixture of organic solvents.

Dyestuffs may be added to these coatings in order to readily distinguish between tablets containing active substances or different amounts of the active compound.

For the preparation of soft gelatin capsules (pearl-shaped closed capsules) consisting of gelatin and, for example, glycerol, or similar closed capsules, the active substance may be admixed with a vegetable oil. Hard gelatin capsules may contain granulates of the active substance in combination with solid, fine grain carriers such as lactose, saccharose, sorbitol, mannitol, starches (e.g. potato starch, corn starch or amylopectin), cellulose derivatives or gelatin.

Liquid preparations for oral application may be in the form of syrups or suspensions, for example, solutions containing from about 0.2% to about 20% by weight of the active substance herein described, the balance being sugar and a mixture of ethanol, water, glycerol and propyleneglycol. Optionally such liquid preparations may contain colouring agents, flavouring agents, saccharine and carboxymethylcellulose as a thickneing agent.

Solutions for parenteral applications by injection can be prepared in an aqueous solution of a water-soluble pharmaceutically acceptable salt of the active substance preferably in a concentration of from about 0.5% to about 10% by weight. These solutions may also contain stablizing agents and/or buffering agents and may conveniently be provided in various dosage unit ampoules.

In therapeutical treatment the suitable daily doses of the compounds of the invention are 100-5000 mg for oral application, preferentially 500-3000 mg, and 0.5-500 mg for parenteral applications, preferentially 25-250 mg.

Working Examples

The following examples will further illustrate the invention.

Example 1. 2-Hydroxy-6-methoxybenzaldehyd (4)

A mixture of 3-methoxyphenol (1) (114.5 g, 0.92 mol), ethylvinyl ether (99.8 g, 1.38 mol), trichloroacetic acid (3.01 g, 0.18 mol) and chloroform (550 ml) was stirred at room temperature for 20 h. The reaction mixture was diluted with ether and extracted twice with 0.5 M NaOH and once with brine. The orgainic layer was dried ($K_2CO_3$) and evaporated. The 1-[(l-ethoxy)-ethoxy]-3-methoxybenzene obtained (2) was litiated without further purification.

BuLi (1.6-M in n-hexan; 76 ml, 0.122 mol) was added dropwise at room temperature during 1 h to a mixture of 1-[(l-ethoxy)]-3-methoxybenzene (2) (12.0 g, 0.061 mol) in dry ether (120 ml) whereupon the mixture was stirred for 2 h under $N_2$. Dimethylformamide (14.3 g, 0.02 mol) in dry ether (25 ml) was added slowly at 10°C during 1 h and stirring was continued for 2 h. The reaction mixture was poured onto crushed ice and extracted 3 times with ether. The organic layer was dried ($Na_2SO_4$) and evaporated to give 2-[(l-ethoxy)-ethoxy]-6-methoxy-benzaldehyd (3) as an oil. 2-M HCl was added dropwise to a stirred mixture of the oil obtained (11.9 g, 53 mmol) in MeOH. The precipitated product 2-hydroxy-6-methoxy--benzaldehyd was filtered and recrystallized from n-hexane. Mp 72-73°C yield 10.6 g (78%).

Example 2. 3-Cyano-5-methoxy-1,2-benzopyran (5)

A mixture of 2-hydroxy-6-methoxybenzaldehyd (4) (1.0 g, 6.6 mmol), acrylonitrile (1.74 g, 33.0 mmol) and DABCO[®] (0.16 g, 1.5 mmol) was refluxed for 2 h. The reaction mixture was diluted with chloroform (10 ml) and extracted twice with saturated $NaHCO_3$, once with diluted HCl and once with $H_2O$. The organic lyaer

was dried ($Na_2SO_4$) and evaporated to afford the desired compound which was recrystallized from EtOH-$H_2O$ (1:1)
Yield: 0.73 g (60%)
M.p.: 70.5-72.0°C

Example 3. 5-Methoxy-1,2-benzopyran-3-carboxylic acid (6)

3-Cyano-5-methoxy-1,2-benzopyran (5) 25.0 g; 0.0133 mol) in 60 ml of 10% aqueous NaOH was refluxed for two hours under a flow of nitrogen. Cooling the solution followed by acidification with hydrochloric acid (6M) gave 25.1 g (92%) of the desired compound. Recrystallization from ethanol-$H_2O$ afforded yellow needles.
M.p. 220-222°C

Example 4. 5-Methoxychroman-3-carboxylic acid (7)

5-Methoxy-1,2-benzopyran-3-carboxylic acid (6) (30 g; 145 mol) was esterified by boiling in ethanol (400 ml) with sulphuric acid (5 ml) as catalyst. The excess of ethanol was evaporated and the ester obtained was extracted with ether. After drying and evaporation 5-methoxy-1,2-benzopuran-3-carboxyethyl ester obtained characterized by NMR and was used in the next step without further purification. A solution of the ester obtained (10% in ethanol) was then hydrogenated over 5% Pd/C at normal pressure and room temperature. When the calculated amount of $H_2$ had been adsorbed, (4 h) the mixture was filtered, the soluent evaporated. The ester was treated with boiling KOH (12 g; 0.213 mol in ethanol/water 50 + 50) for 1 hour. By acidification of the alcaline solution the saturated acid (7) was isolated as white chrystals in 60% overall yield (24 g).
M.p. 171-173°C (from ethanol-$H_2O$)

Example 5. 3-amino-5-methoxychroman (8)

A mixture of 5-methoxy-chroman-3-carboxylic acid (7) (10.0 g; 0.048 mol), diphenylphosporazidate (16.6 g; 0.0603 mol), triethylamine (6.10 g; 0.0603 mol) in benzene (150 ml) was stirred under reflux for 2 hours. Benzylalcohol (6.52 g; 0.0603 mol) was added and the reflux was continued for further 24 hours. The mixture was evaporated and the residue was dissolved in toluene. The solution was successively washed with 5% citric acid, water, saturated $NaHCO_3$ and brine. Drying and evaporation gave an oil which crystallized from an ether solution. The crystilline 3-benzyloxycarbonyl-5-methoxychroman (6 g; 0.0192 mol) was dissolved in methanol. A solution of hydrochloric gas in methanol (33 ml; 0.58-M) and a catalyst 5% Pd/C (3 g) were added, the mixture was hydrogenated at room temperature and athmospheric pressure (3 h). After filtration and evaporation the remaining product is a colourless viscous oil. The product was dissolved in water and after alkalization the free base was extracted with methylene chloride. After drying and evaporation of the methylene chloride solution, the oil was converted to its hydrochloride and was recrystallized from ethanol-ether.
M.p 237-38°C. Yield 6.0 g (70%).

Example 6. 5-Methoxy-3-propylaminochroman (9)

To a solution of 3-amino-5-methoxychroman (8) 2.0 g; 0.028 mol) in dimethyl formamide (10 ml) was added potassium carbonate (11.6 g; 0.084 mol) and a catalytic amount of sodium iodine. l-Bromo-propane (2.05 g, 0.017 mol) was added dropwise at 50°C with stirring. Stirring was continued for 5 hours at 50°C. The mixture was cooled and diluted with water (300 ml). The solution was extracted twice with ether. After drying ($Na_2SO_4$) and evaporation of the organic solvent the desired product was obtained in 89% yield (6.6 g). The product was precipitated as the hydrochloride salt and recrystallized from ethanol acetonitrile.
M.p. 194-195°C.

Example 7. 3-dipropylamino-5-methoxychroman (11)

This compound was obtained from 3-amino-5-methoxychroman (8) (2.8 g; 0.0156 mol) in the same way as described in Example 6.
Yield: 3.28 g as base (80%). Its hydrochloride salt had mp: 137-139°C (from ethanol-ether)

Example 8. 5-Hydroxy-3-propylaminochroman (10)

A solution of BBr (500 mg; 1.0 mmol) in $CH_2Cl_2$ was added dropwise to an icecooled solution of 5-methoxy-3-propylaminochroman (9) (150 mg; 0.569 mmol) in $CH_2Cl_2$ (20 ml) over 10 min. Stirring was continued for additional 1 hour at 0°C. The mixture was poured on ice, and NaCl concentraded $NH_4OH$ and NaCl and was extracted twice with $CH_2Cl_2$. The collected organic phases were dried and evaporated. The residual colourless oil was made crystalline as the hydrochloride salt.
Yield 90 mg (76%) (from ethanol-ether)
M.p. 172-174°C

Example 9. 3-dipropylamino-5-hydroxychroman (12)

This compound was prepared from 3-dipropylamino-5-methoxychroman (11) (1.85 g; 0.007 mol) in the same was as described in Example 8. The demethylation was complete after 5 minutes of stirring at 0°C. Yield: 1.5 g as base (86%). Its hydrochloride salt had m.p. 208-210°C (from acetonitrile)

Example 10. (-)-3-amino-5-methoxychroman (13)

To racemic 3-amino-5-methoxy chroman (8) (12 g, 0.067 mol), dissolved in 150 ml of methanol, was added an equivalent amount of L(+) tartaric acid, dissolved in 150 ml of methanol. The preciptitate was filtered off and recrystallized 5 times from water to give 3.5 g of a tartrate with 97% optical purity of the (-)isomer. Alkalization, extraction with ($CH_2Cl_2$) drying ($Na_2SO_4$) and evaporation gave 1.9 g (16%) as the free base of (13) $[\alpha]_D^{25}$ = 20.8° [C = 0.833, $CHCl_3$].

Example 11. (+)-3-amino-5-methoxychroman (14)

The mother liquors from (13) were collected and extracted to free base. 8 g was received with an optical purity of 65% (+). The tartrate was prepared by adding the base dissolved in methanol to the equivalent amount of D(-)-tartaric acid in water. The precipitate was filtered off and further three recrystallizations gave 100% optical pure compound (14) as free base
Yield: 1.5 g $[\alpha]_D^{25}$ = +25.0° [C = 2.0:$CH_2Cl_2$].

Alkylation and demethylation of compounds (13) and (14) were achieved according to the methods for compounds (11) and (12) yielding the two enantiomers (15) and (16).

Example 12. (+)-3-di-propylamino-5-hydroxychroman hydrochloride (23)

The hydrochloride was prepared by adding HCl-ether (3M) to the base (16) dissolved in acetonitrile.
Yield: 1.2 g (74%) of compound (22) as white crystals.
M.p. 237-238° (decomposition $[\alpha]_D^{25}$ = +22.8° [C = 0.5. methanol].

Example 13. (-)-3-di-propylamino-5-hydroxychroman hydrochloride (24)

Prepared from the (-) base (15) as (23) gave.
Yield: 1.0 g (71%) as white crystals.
M.p. 235-236° (decompostion $[\alpha]_D^{25}$ = -23.0 [C = 0.5. methanol].

Example 14. 3-ethylpropylamino-5-methoxychroman (17)

A mixture of 3-N-ethylpropylamino-5-methoxychroman (0.7 g; 3.2 mmol), bromoethane (1.0 g; 8.1 mmol), $K_2CO_3$ (2.9 g; 14 mmol), DMF (15 ml) and catalytic amount of KI was stirred at 70° over night. The product was added 100 ml of ether and washed with 5x25 ml of water. Flash chromatography ($SiO_2$, diisopropylether) gave 0.5 g (63%) of compound (17) as a light brown oil. GC indicated 98% purity. The hydrochloride did not crystallize why it was used directly in the demethylation step.
MS (70 eV): m/z 249 48% M-29 100%.

Example 15. 3-ethylpropylamino-5-hydroxychroman (18)

0.6 g (2 mmol) of 3-N-ethylpropylamino-5-methoxychroman hydrochloride was dissolved in $CH_2$-$Cl_2$ and added 5 ml (5 mmol) of 1 M $BBr_3$ solution in $CH_2Cl_2$. Conventional work-up gave 0.4 g (80%) of compound (18) as a colourless oil. The hydrochloride was crystallized from $CH_3CN$ to yield 0.3 g (60%) white crystals. M.p. 194-196° (decomposition).

Example 16. 3-n-propylphenethylamino-5-methoxychroman (19)

A mixture of 3-amino-5-methoxychroman (0.5 g; 3 mmol), (2-Bromoethyl)-bensene (0.5 g; 3 mmol), $K_2CO_3$ (1 g; 7 mmol) and DMF (3.5 ml) was stirred at 70° over night. GC indicated 100% of a new product, work-up gave 0.8 g of a brown oil. The crude oil was added 1 g of l-bromopropane, 1 g of $K_2CO_3$, 3 ml of DMF and catalytic amounts of KI. The mixture was stirred at 70°. Extracting and flash chromatography ($SiO_2$, Diisopropylether) gave 0.5 g of compound (14) as a colourless oil with 98% purity on GC.
MS CI (70 eV): m/z +1 326 (100%), 234 (7%).

Example 17. 3-propylphenethylamino-5-hydroxychroman (20)

The oil (5) (0.5 g 0.0015 mol) was dissolved in ether and added 3.5 M HCl in ether in excess. An oil was formed. The ether was distilled off and the oil was dissolved in $CH_2Cl_2$ (3 ml) and 4.5 ml (0.0045 mol) of 1 M $BBr_3$ in $CH_2Cl_2$ was added.
Yield: 0.2 g (40%) of compound (20) as hydrochloride in form of white crystals.
MS EI (70 eV): 220 (m/z-91) (100%)
M.p. 258-260°.

Example 18. 3-(4-fluorobenzyl)propylamino-5-methoxychroman (21)

0.5 g (0.002 mol) of 3-propylamino-5-methoxychroman hydrochloride was mixed with 0.5 g (0.003 mol) of 4-fluorobenzylbromid, 3 ml of DMF, 0.7 g (0.005 mol) $K_2CO_3$ and catalytic amounts of KI. The mixture was stirred at 70° for 6 hours. Yield after work-up gave 0.7 g of a light brown oil (21). GC indicates 100% purity. The oil as directly used without any further purification in the demethylation step.

Example 19. 3-(4-fluorobenzyl)propylamino-5-hydroxychroman (22)

Demethylation according to standard methods yield 0.4 g (63%) of a colourless oil with a purity of 100% according to GC.
MS EI (70 eV): m/z 315 (12%), 109 (100%), 286 (29%).

Example 20. 3-butylpropylamino-5-methoxychroman (23)

A mixture of 5-methoxy-3-propylaminochroman (9) (0.43 g; 1.9 mmol), iodobutane (0.54 g; 2.9 mmol), $K_2CO_3$ (0.54 g; 3.9 mmol) and DMF (1 ml) was stirred at 50°C for 26 h. The mixture was added 20 ml of $H_2O$ and extracted 3 times with ether (dried and evaporated) Chromatotron chromatography ($SiO_2$; chloroform/n-hexane/ethanol/$NH_3$ (aq), 60:38:2:0.05) gave 0.51 g (95%) of compound (23) as an oil. GC indicated 98% purity and NMR confirmed the correct structure. The base was used directly in the demethylation step.

Example 21. 3-butylpropylamino-5-hydroxychroman (24)

0.49 g (1.8 mmol) of 3-butylpropylamino-5-methoxychroman was dissolved in $CH_2Cl_2$ (50 ml) and $BBr_3$ - (4.9 ml; 5.3 mmol) in $CH_2Cl_2$ (55 ml) was added to an ice-cooled solution of 3-amino-butylpropyl-8-methoxychroman in $CH_2Cl_2$ (50 ml). After 3 h stirring a conventional work-up gave 0.48 g (100%) of the desired compound (24) as a brown oil. The hydrochloride was recrystallized from isopropanol/diisopropylether to yield 0.49 g.
mp. 166.9 - 168.1°C.

Pharmacology

Pharmacological treatment of depression in man

Evidence is available that in depressed patients the neurotransmission in the central nervous system (CNS) may be disturbed. These disturbances appear to involve the neurotransmitters noradrenaline (NA) and 5-hydroxytryptamine (5-HT). The drugs most frequently used in the treatment of depression are considered to act by improving the neurotransmission of either or both of these physiological agonists. Available data suggest that the enhancement of 5-HT neurotransmission will primarily improve the depressed mood and anxiety, whereas the enhancement of noradrenaline neurotransmission will rather improve the retardation symptoms occurring in depressed patients. In recent years may efforts have been made to develop new drugs with high selectivity for the improvement of the 5-HT neurotransmission in the CNS.

The mechanism of action for the drugs generally used today in the therapy of mental depression is indirect, i.e. they act by blocking the reuptake of the neurotransmitters (NA and/or 5-HT) released from nerve terminals in the CNS, thus increasing the concentration of these transmitters in the synaptic cleft and hence restoring an adequate neurotransmission.

A fundamental different way to improve the neurotransmission in the central 5-HT-neurons would be to use a direct 5-HT-receptor agonist. In order to minimize side effects, a high selectivity for this kind of receptors would then be preferable.

Surprisingly, we have found that a group of compounds of the formula I have selective, direct stimulating effect on central 5-HT receptors. In order to evaluate the 5-HT-receptor stimulating effect and selectivity, the effects on various receptors in rat brain was measured in vitro using receptor assays ($IC_{50}$mM).

In vitro test: Receptor binding assay

Method

The method of Peroulka & Snyder (Mol.Pharmacol. 16, 687-699, 1979) was used. Male Sprague-Dawley rats weighing 150-200 g were decapitated, and their brains were rapidly removed. The striata were dissected, pooled and homogenized in 50 mM Tris-HCl buffer (pH 7.6). The membrane fraction was collected by centrifugation (48 000 g for ten minutes), washed once with the buffer, and resuspended in 50 mM Tris HCl (pH 7.6) containing 0.1% ascorbic acid, 10 mM paragyline, 120 mM NaCl, 5 mM KCl, 2 mM $CaCl_2$ and 1 mM $MgCl_2$. The suspension was preincubated at 37°C for 10 minutes and then kept on ice until use.

The assays have been carried out using a cell harvester equipment. The incubations were made in

quadruplicate, each well containing membrane suspension, $^3$H-spiperone (0.4 nM) or $^3$H-5-HT (5 nM) and the test compound in a final volume of 0.5 ml. After incubation for 10 minutes at 37°C, the contents of the wells were rapidly filtered and washed on Whatman GF B filters using the Cell harvester. The specific binding was defined as the difference of radioligand bound in the presence and in the absence of a specific displacer (d-LSD, lμM and 5-HT, 10 μM for $^3$4-spiperone and $^3$H-5-HT, respectively). The test results are expressed as $IC_{50}$. The $IC_{50}$ value is given in nM, indicates the concentration of the test substance which reduces the amount of specifically bound radioligand by 50%..

Test Result

3-di-propylamino-5-hydroxychroman was inactive in $^3$H-spiroperidol (Dopamine-$D_2$) and $^3$H-Ketanserin (5-$HT_2$) receptor binding studies, while it was active in $^3$H-5-HT (5-HT ), showing a $IC_{50}$ 0.083 μM.

This pharmacological data affirm that the aminochromans of the present invention are very potent centrally acting 5-HT-receptor stimulating agents with high selectivity for certain 5-HT receptors over DA and NA receptors. Due to this unique pharmacological profile, the compounds are of great clinical interest in the treatment of certain disorders in the CNS such as depression and anxiety. Additional potential uses of the new compounds are in control of pain, where 5-HT has been shown to be involved, and in senile disturbances of movement and mental function e.g. senile dementia, where a decreased level of brain 5-HT has been demonstrated.

Best mode of carrying out the invention

The compounds 3-(di-propylamino)-5-hydroxychroman and 3-ethylpropylamino-5-hydroxychroman and their optical enantiomers and salts, processes for preparing said compounds and methods employing said compounds in therapy represent the best mode of carrying out the invention known to the inventors at present.

## Claims

1. A compound of the formula

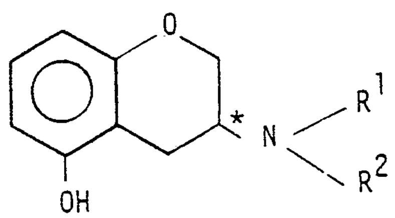

I

wherein R' is a saturated or unsaturated alkyl group having 1-5 carbon atoms or a phenylalkyl group, whereby the phenyl ring may be substituted or unsubstituted and the alkyl having 1-4 carbon atoms, $R^2$ is hydrogen or an alkyl group having 1-5 carbon atoms, or R' and $R^2$ together form a 5-or 6-membered ring containing 1 or 2 heteroatoms selected from N, O and S, and enantiomers and physiologically acceptable salts thereof.

2. A compound according to claim 1 wherein R' and $R^2$ are the same or different and selected from ethyl and n-propyl.

3. 3-dipropylamino-5-hydroxychroman, enantiomers and physiologically acceptable salts thereof.

4. A process for the preparation of a compound of the formula I according to claim 1, by

a) cleaving of an ether of the formula II

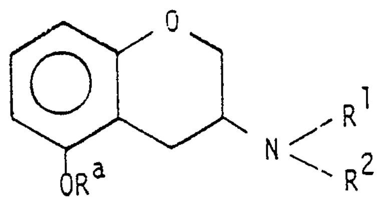

II

wherein $R^a$ represents a hydrocarbon residue, and $R^1$ and $R^2$ are as defined under formula I, to the formation of a compound of formula I,

b) N-alkylation of a compound of formula III

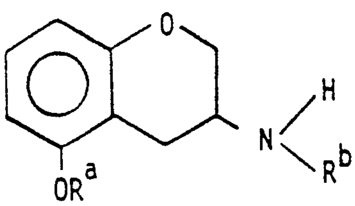

III

wherein $R^a$ is as defined under formula II, $R^b$ is either $R^1$ or $R^2$, whereby $R^1$ and $R^2$ are as defined under formula I, into a compound of formula I with an appropriate alkylating agent,

c) reduction of a compound of the formula IV

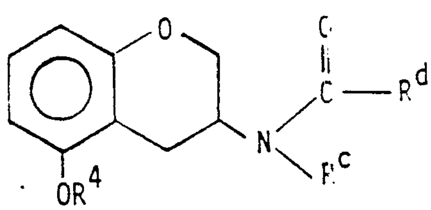

IV

wherein $R^4$ is hydrogen or $R^1$, $R^c$ is hydrogen or $R^1$ whereby $R^1$ is defined under formula I and $R^d$ is hydrogen or an alkyl group having 1 to 4 carbon atoms to the formation of a compound of formula I with an appropriate reducing agent,

d) catalytical reduction of a compound of the formula V

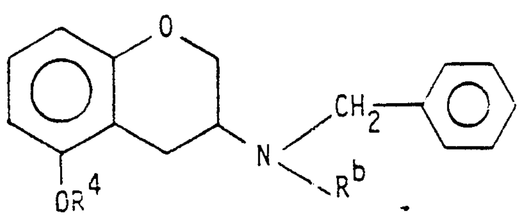

V

wherein $R^4$ and $R^b$ are as defined above to the formation of a compound of formula I,

e) converting a compound of the formula VI

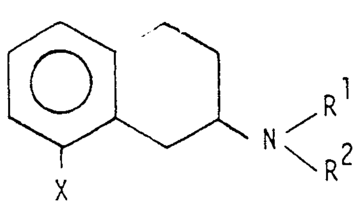

VI

wherein X represents $SO_3H$, $NH_2$ or Cl and $R^1$ and $R^2$ are as defined under formula I by substitution of the group X to a hydroxy group to formation of a compound of formula I.

f) converting of an enamine with either a $C_1$-$C_2$ or a $C_2$-$C_3$ double bond or an imine base (without $R^2$) or immonium salt (e.g. $ClO_4$ or $BF_4$ ) with a $C_2$-N double bond of the formula

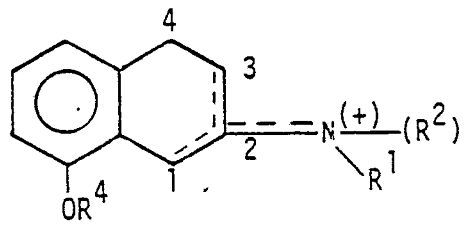

VII

wherein $R^4$, $R^1$ and $R^2$ are as defined above by reduction to a compound of formula I or Ia, preferably by catalytic hydrogenation, whereupon optionally a base obtained is converted to a physiologically acceptable acid addition salt or a salt obtained is converted to the base or to a different, physiologically acceptable acid addition salt, and optionally an isomeric mixture obtained is separated to a pure isomer.

5. A pharmaceutical preparation containing as active ingredient a compound according to any of claims 1-3, an enantiomer or a physiologically acceptable salt thereof.

6. A method for treatment of disorders in the central nervous system, especially 5-hydroxytryptamine mediated disorders by administering to mammals and a man compound defined in claims 1-3, an enantiomer or a physiologically acceptable salt thereof.

7. A method according to claim 6 for treatment of depression, anxiety, anorexia, hypertension, senile dementia, termoregulator and sexual disturbances.

8. A method according to claim 6 for treatment of pain.

9. A compound as defined in any of claims 1-3, an enantiomer or a physiologically acceptable salt thereof, for use in therapy.

10. A compound according to claim 9 for use in the treatment of depression, anxiety, anorexia, senile dementia, hypertension, termoregulator and sexual disturbances.

11. A compound according to claim 9 for use in the treatment of pain.

12. Use of a compound according to any of claims 1-3 for the manufacture of a medicament for treatment of disorders in the central nervous system, especially 5-hydroxy tryptamine medicated disorders.

13. Use according to claim 12 for the manufacture of a medicament for treatment of depression, anxiety, anorexia, senile dementia, hypertension. termoregulator and sexual disturbances.

14. Use according to claim 12 for manufacture of a medicament for treatment of pain.

15. A compound of the formula

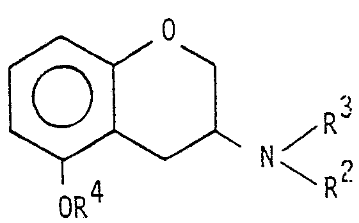

IA

wherein $R^4$ is hydrogen, saturated or unsaturated alkyl containing 1-5 carbon atoms or a phenylalkyl whereby the phenyl ring may be substituted or unsubstituted and the alkyl having 1-4 carbon atoms,
$R^3$ is hydrogen or saturated or unsaturated alkyl containing 1-5 carbon atoms or an phenylalkyl, whereby the phenyl ring may be substituted or unsubstituted and the alkyl having 1-4 carbon atoms.
$R^2$ is hydrogen or alkyl containing 1-5 carbon atoms or $R^2$ and $R^3$ together form a 5-or 6-membered ring containing 1 or 2 heteroatoms selected from N. O and S
with the proviso that when $R^4$ is hydrogen then $R^3$ is hydrogen. enantiomers and salts thereof.

16. A compound according to claim 15 wherein $R^4$ is methyl.

17 A compound according to claims 15-16 wherein $R^2$ and $R^3$ are the same or different selected from ethyl and n-propyl.

18. A pharmaceutical preparation containing as active ingredient a compound according to claim 15. an enantiomer or a physiologically acceptable salt thereof.

19. A method for treatment of disorders in the central nervous system. especially 5-hydroxytryptamine mediated disorders by administering to mammals and man a compound defined in claim 15. an enantiomer or a physiologically acceptable salt thereof.

20. A method according to claim 15 for treatment of depression, anxiety, anorexia, hypertension, senile dementia, termoregulator and sexual disturbances.

21. A method according to claim 15 for treatment of pain.

22. A compound as defined in claim 15, an enantiomer or a physiologically acceptable satl thereof, for use in therapy.

23. A compound according to claim 15 for use in the treatment of depression, anxiety, anorexia, senile dementia, hypertension, termoregulator and sexual disturbances.

24. A compound according to claim 15 for use in the treatment of pain.

25. Use of a compound according to claim 15 for the manufacture of a medicament for treatment of disorders in the central nervous system, especially 5-hydroxy tryptamine medicated disorders.

26. Use according to claim 15 for the manufacture of a medicament for treatment of depression, anxiety, anorexia, senile dementia, hypertension, termoregulator and sexual disturbances.

27. Use according to claim 15 for manufacture of a medicament for treatment of pain.

28. Compounds, processes, pharmaceutical preparations, use of and methods for treatment as claimed in any of claims 1-27 and substantially as described.

European Patent Office

# PARTIAL EUROPEAN SEARCH REPORT

which under Rule 45 of the European Patent Convention shall be considered, for the purposes of subsequent proceedings, as the European search report

Application number
EP 87850388.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | SE, B, 340 286 (PARKE, DAVIS & COMPANY)<br>--- | 1,15 | C 07 D 311/58 |
| A | EP,A1, 0 027 835 (WARNER-LAMBERT COMPANY)<br>--- | 1,15 | A 61 K 31/35 |
| P,X | EP,A2, 0 222 996 (CIBA-GAIGY AG)<br>*The whole document, especially example 10*<br>--- | 1-5<br>9-18<br>22-28 | |

TECHNICAL FIELDS SEARCHED (Int. Cl.4)

C 07 D

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 2-5, 9-14, 16-18, 22-28

Claims searched incompletely: 1,15*)

Claims not searched: 6-8,19-21

Reason for the limitation of the search:

Methods for treatment of the human or animal body by surgery or therapy, as well as diagnostic methods. (See art. 52(4) of the European Patent Convention)

*)The wording "$R^1$ and $R^2$ ($R^2$ and $R^3$)together form a 5- or 6-membered ring containing 1 or 2 heteroatoms selected from N, O or S" is too broadly formulated to permit a meaningful search. The search on claims 1och 15 has been incomplete.

| Place of search | Date of completion of the search | Examiner |
|---|---|---|
| Stockholm | 21-03-1988 | ELFVING L. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03 82